# EUROPEAN PATENT APPLICATION

(11) **EP 3 647 912 A1**
(43) Date of publication of application: **06.05.2020**
(21) Application number: 18461622.5
(22) Date of filing: 05.11.2018
(51) Int. Cl.: G06F 3/01, A61F 2/50, A61F 2/68

(54) **A SYNAESTHETIC SYSTEM AND A METHOD FOR SYNESTHESIA**

(71) Applicant: VBIONIC Sp. z o.o., 60-612 Poznan (PL)
(72) Inventor: Rajewski, Bartosz, 60-612 Poznan (PL); Luczak, Adam, 61-674 Poznan (PL)
(74) Representative: Kancelaria Eupatent.pl Sp. z.o.o

(57) **Abstract**

One of the inconveniences for patients that underwent amputations is a lack of ability of sensing tactile stimuli in the part of a limb they lost. This invention proposes a solution consisting in converting tactile stimuli into sound stimuli. This is done by means of a tactile sensor placed at the distal part of a prosthesis, whose signal converted appropriately determines generating a sound reflecting the touched surface. The sound can be defined for particular textures of the surface or modulated directly by the sensor signal. The system allows for detecting contact with an object or the surface and for extending perceptual skill of a non-amputee as well.

## Description

### TECHNICAL FIELD

The present invention relates to a synaesthetic system and method for synesthesia. In particular, the present invention relates to the synaesthetic system comprising at least one tactile sensor and a central processing unit, capable of converting signals from at least one tactile sensor into audio signals possible to be listened to by a user.

### BACKGROUND OF THE INVENTION

It is commonly believed that people have five senses: vision, hearing, taste, smell, and touch, however, all of them have their limitations. Vision registers only a certain electromagnetic wavelength range, whereas hearing has limitations concerning sound frequency, etc. However, such an arrangement allows a person to interpret reality in a particular way.

In case of losing one of the senses, a certain area of information reaching the brain from the outside world is lost and perception changes. It influences the increase in effectiveness of other senses as well. In case of people who underwent a partial amputation of the upper limb, a lack of ability of sensing touched and held objects is one of the problems.

The solutions that aid sensing external stimuli already exist: in the patent US 2010/0286571 A1, in which leaning the body is reflected in vibrations in the other body part, whereas in the patent US 2008/0200994 A1 sensing force, vibrations and temperature from the end of prosthesis can be reflected in the other place on skin.

None of the above however aim at partial restoration of perceptual skill, namely a possibility of sensing touch after undergoing a partial amputation of the upper limb in the other way. A sense affected by external stimuli makes here the difference with regard to proper functioning of the human organism.

The aim of the development of the present invention is an improved synaesthetic system and method for synesthesia.

### SUMMARY AND OBJECTS OF THE PRESENT INVENTION

The object of the invention is a synaesthetic system for converting a signal caused by contact or movement across surfaces into a sound, the system being characterized in that it comprises: at least one tactile sensor operated by a tactile sensors module; an audio responses generator configured to generate audio responses according to signals received from said tactile sensor; a controller configured to receive data from said at least one tactile sensor; receive an audio response from said audio responses generator; and output said audio response.

Preferably, said at least one tactile sensor is a piezoelectric sensor.

Preferably, said audio responses generator comprises: a materials database comprising data identifying materials known to the system and signal parameters with which such materials respond to the at least one tactile sensor; a sounds database comprising data identifying audio responses; a memory configured to store a correspondence between a surface material, from said materials database, and an audio clip, from said sounds database; wherein said controller configured to receive data from said at least one tactile sensor and identify presence of a correspondence between said data and a material from said materials database; and in case such correspondence is found, to output an identified audio response.

Preferably, said materials database comprises, for each material's signal sample, an average voltage, a minimum voltage, a maximum voltage, standard deviation as well as a number of times the voltage crosses zero.

Preferably, said outputting an identified audio response if effected by a wireless communication to a headphones unit.

Preferably, said audio clip is output using a Bluetooth communication.

Preferably, at least one tactile sensor is positioned on a prosthetic.

Preferably, said system has a shape of a glove, comprising said tactile sensor at a tip of at least one finger of said glove wherein said central processing unit is sewn in at the wrist of the glove.

Preferably, said system is integrated into a prosthesis.

Preferably, said audio response is modulated by the data from said at least one tactile sensor.

Preferably, said modulation process comprises normalization of said data within a predefined range of sound parameters.

Another object of the invention is a synaesthetic method for converting a signal caused by contact or movement across surfaces into a sound, the method being characterized in that it comprises the steps of: providing a reference database comprising signal patterns of different materials; associating different signal patterns with different audio clips; reading data from at least one tactile sensor and comparing the read data with said signal patterns; wherein when a match is found outputting a corresponding audio clip to a user via an audio module.

Another object of the present invention is a computer program comprising program code means for performing all the steps of the computer-implemented method according to the present invention when said program is run on a computer.

Another object of the present invention is a computer readable medium storing computer-executable instructions performing all the steps of the computer-implemented method according to the present invention when executed on a computer.

### BRIEF DESCRIPTION OF THE DRAWINGS

These and other objects of the invention presented herein, are accomplished by providing a synaesthetic system and method for synesthesia. Further details and features of the present invention, its nature and various advantages will become more apparent from the following detailed description of the preferred embodiments shown in a drawing, in which:
Fig. 1 is a schematic representation of practical application of synaesthetic system;
Fig. 2 is a schematic representation of movement of a prosthesis with a sensor across the surface of different roughness;
Fig. 3 shows diagrams presenting unprocessed responses from the piezoelectric sensor after moving it across specific surfaces;
Fig. 4 presents a diagram of the system according to the present invention; and
Fig. 5 presents a diagram of the method according to the present invention.

### NOTATION AND NOMENCLATURE

Some portions of the detailed description which follows are presented in terms of data processing procedures, steps or other symbolic representations of operations on data bits that can be performed on computer memory. Therefore, a computer executes such logical steps thus requiring physical manipulations of physical quantities.

Usually these quantities take the form of electrical or magnetic signals capable of being stored, transferred, combined, compared, and otherwise manipulated in a computer system. For reasons of common usage, these signals are referred to as bits, packets, messages, values, elements, symbols, characters, terms, numbers, or the like.

Additionally, all of these and similar terms are to be associated with the appropriate physical quantities and are merely convenient labels applied to these quantities. Terms such as "processing" or "creating" or "transferring" or "executing" or "determining" or "detecting" or "obtaining" or "selecting" or "calculating" or "generating" or the like, refer to the action and processes of a computer system that manipulates and transforms data represented as physical (electronic) quantities within the computer's registers and memories into other data similarly represented as physical quantities within the memories or registers or other such information storage.

A computer-readable (storage) medium, such as referred to herein, typically may be non-transitory and/or comprise a non-transitory device. In this context, a non-transitory storage medium may include a device that may be tangible, meaning that the device has a concrete physical form, although the device may change its physical state. Thus, for example, non-transitory refers to a device remaining tangible despite a change in state.

As utilized herein, the term "example" means serving as a non-limiting example, instance, or illustration. As utilized herein, the terms "for example" and "e.g." introduce a list of one or more non-limiting examples, instances, or illustrations.

### DESCRIPTION OF EMBODIMENTS

One of the problems for people who underwent the upper limb amputation is inability of sensing tactile stimuli from a prosthesis. When a patient holds an object and does not look at a prosthesis, such patient does not know exactly what is being held or touched i.e. said patient can only assess the weight of an object loading the residual limb.

The present invention allows for partial restoration of the ability to sense. It concerns receiving information in a form of a certain sound that informs on the texture of the touched surface.

Differentiating touched surfaces that vary in roughness or, if possible, in other material features allows for that. It is determined by an output signal from a piezoelectric or other tactile sensor, which processed by means of dedicated methods, is information required to generate a specific audio signal.

The sound can be played in real time and take various forms. The defined tunes can be assigned to the defined surfaces by default, however, a possibility of adjusting the sound to particular surfaces by a user is allowed. For unknown surfaces, the sound can be the reflection of the texture by modulations of the sound of default frequency.

According to the present invention, it is a sound stimulus reaching the user via the headphones or a loudspeaker. A person using the invention can sense the texture of a material by means of the sound, for example, stroking a cat, a user can hear a certain pleasant sound, certainly only if such a person is not allergic to cats, otherwise the synaesthetic system can generate, for example, the unpleasant sound. This shows possibility of personalizing the system.

The invention allows for assigning the sound characteristics to a contact with particular materials. It also allows for analyzing information from the sensor in contact with touched objects. By means of special methods, a patient can sense the surrounding world from a bit broader perspective, in which a tactile stimulus is converted into a sound.

The numbers in the following detailed description of the invention are based on assigning a particular number to a specific element. Not all the numbered elements are strictly connected with the invention; some of them are intended to explain the operation mode. It is important, therefore, not to assign strictly the subject matter of the invention to the presented pictures, but only to understand the operation mode and possible construction of the invention.

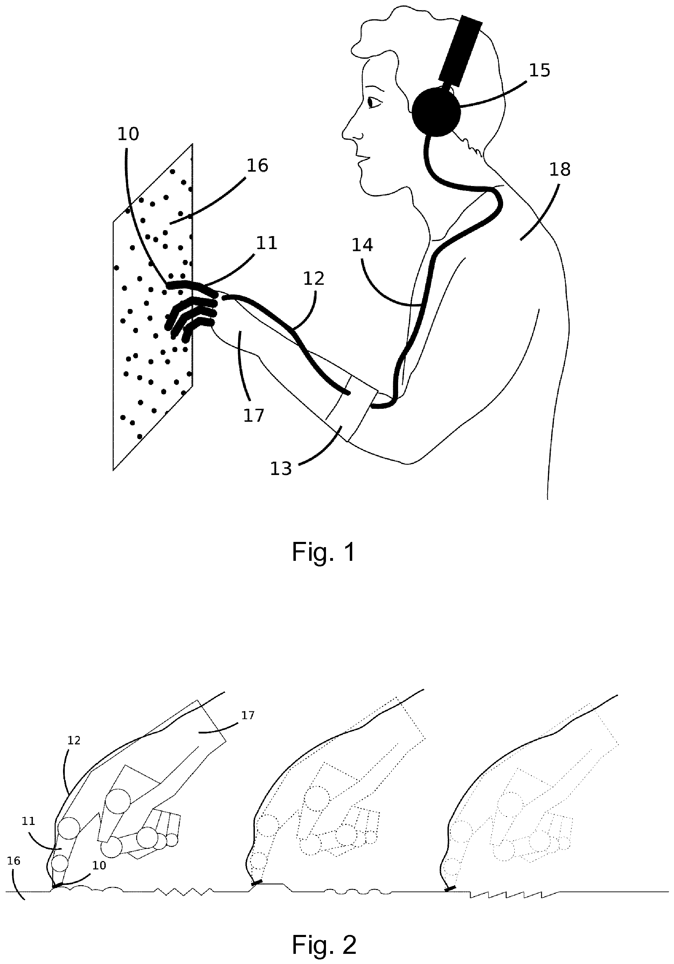

Fig. 1 is a schematic representation of practical application of synaesthetic system for sensing the tactile stimuli based on signals from at least one tactile sensor 10. A user 18, with their prosthesis 17 on, touching the surface 16 (for example, a wall or flat surface) of a specific texture is able to hear a particular sound, reflecting the texture of the touched surface, via the headphones 15. It can be a continuous or intermittent sound, or a tune set by default or selected by a user.

The sound is generated when the user 18 touches, moves or maneuvers across a particular surface or object, as the tactile sensor 10 contacts this surface or object.

Various surface 16 roughness or other features of the touched material allow for differentiating them while reading the tactile sensor 10. When an action is carried out, information on this movement is sent in real time, for example via a cable 12, to a central processing unit 13, for example worn on a wrist or an arm or integrated within said prosthesis 17.

The system may be implemented as a glove, comprising said tactile sensor at a tip of at least one finger of said glove. Said central processing unit 13 may be sewn in at the wrist of the glove. Such glove is to be worn on a prosthesis 17.
In the central processing unit 13, the signal is converted by means of the algorithm (implemented as hardware, software or a combination of both) into audio information, which is received via the cable 14 by the headphones 15.

Along with the technological development it is possible to omit sending information via the cable 12, 14, adapting a wireless solution, for example, via Bl uetooth.

Fig. 2 presents situations in which a user moves their prosthetic finger 11 with the tactile sensor 10 across a non-smooth surface 16. By means of the signal from a possible to use piezoelectric tactile sensor 10, the system allows for detecting the irregularities of the surface 16. The signal is the response to changeable pressure on the piezoelectric element while moving the prosthesis across the surface 16. Depending on the roughness of the surface 16, the signal is different, which, as a result, allows for defining a group of materials and assigning particular sounds to them.

### Identifying textures

The signal from the tactile sensor 10 placed on a prosthetic finger 11 while touching and also moving across the objects or surfaces 16, is fed to the central processing unit 13, where it is processed.

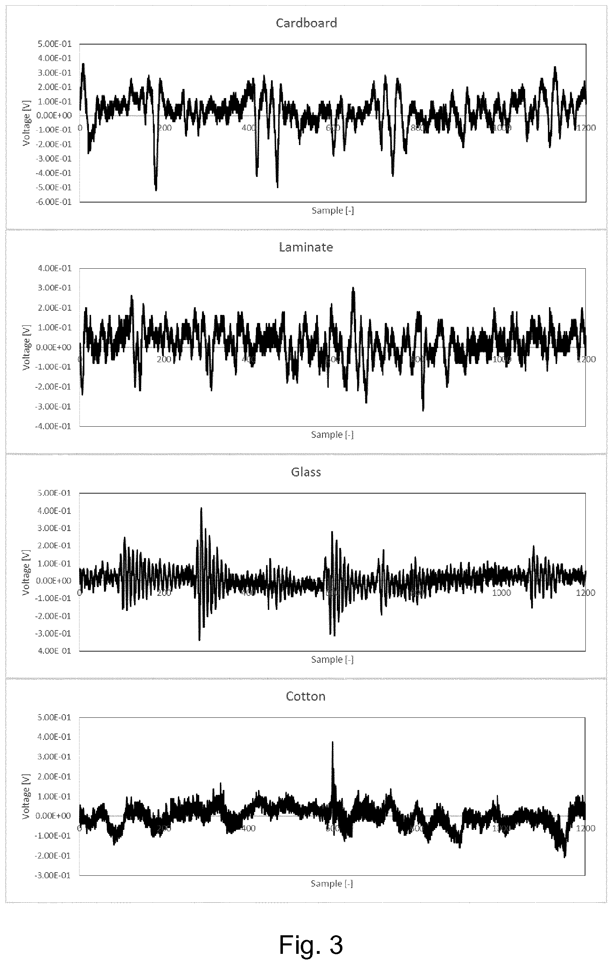

The differences between the sensor signals are shown in Fig. 3, in which each diagram corresponds to moving the finger across one of four surfaces. A scheme in which voltage [V] being a sensor signal is assigned to the vertical axis, whereas the number of sample, that is an individual measurement- to the horizontal axis, was adopted for each diagram.

The diagrams were obtained by means of moving the tactile sensor 10 across selected materials. During the trial, the tactile sensor 10 maintained contact with the surface 16 of a particular material. The tactile sensor 10 response was measured on four different materials, namely: A - cardboard, B - laminate, C - glass, D - cotton. After the first glance, it can be concluded that the tactile sensor 10 responses differ.

Such measurements may be stored in memory in a form of a table comprising an average voltage of a sample, a minimum voltage of a sample, a maximum voltage of a sample, standard deviation as well as a number of times the voltage crosses zero volts within a standardized sample. Table 1 and table 2 below show examples of such measurements. For example, the measurements can be made with a 20 µs periods.

**Tab. 1. Raw signal parameters**

| | cardboard | laminate | glass | cotton |
|---|---|---|---|---|
| Average [V] | 0,027 | 0,020 | 0,007 | -0,001 |
| MIN [V] | -0,520 | -0,320 | -0,336 | -0,208 |
| MAX [V] | 0,360 | 0,300 | 0,416 | 0,376 |
| Standard deviation | 17,686 | 9,998 | 6,879 | 4,405 |
| Number of times of crossing 0 [V] | 336 | 404 | 465 | 524 |

**Tab. 2. Parameters for absolute values**

| | cardboard | laminate | glass | cotton |
|---|---|---|---|---|
| Average [V] | 0,095 | 0,075 | 0,057 | 0,048 |
| MIN [V] | 0,000 | 0,000 | 0,000 | 0,000 |
| MAX [V] | 0,520 | 0,320 | 0,416 | 0,376 |
| Standard deviation | 7,712 | 3,760 | 3,059 | 1,671 |

On the basis of this difference, material surfaces can be defined and particular sounds can be assigned to them. In case of similar sensor responses, a similar sound may be assigned.

### Defining sounds

The kinds of sounds that carry information on a particular surface can be various. The aim is that a particular sound carries information on a particular surface or the one similar to it with its parameters. For example, in case of moving the finger with the tactile sensor 10 across a cotton-made surface 16, the sound assigned to cotton or a category of fabrics, which comprises various woven fabrics such as, for example, linen, polyester, nylon or even to a broader category, which smooth surfaces may form, is generated.

Taking the sounds into consideration, they can be more and less complex; it can be a tune or a sound modulated by means of a converted sensor signal or a single tune or a sound, all of these options can be available to a user at choice from a display interface or a smartphone application.

In case a sound is modulated by means of a converted sensor's signal, parameters of the signal are mapped to sound signal parameters. The minimum sound parameters must be audible for a human while the maximum sound parameters shall not be unpleasant or too loud for a person. Such sound level is preferably between 10 dB (assigned to lowest tactile sensor's 10 readings) and 90 dB (assigned to highest tactile sensor's 10 readings) and in a particular embodiment between 40 dB and 60 dB. Alternatively, a user may set said minimum and maximum sound parameters. This process may be called normalization within a predefined range of sound parameters such as volume levels.

Regarding a huge number of surface features, moving the finger across the unidentified materials can generate in the system sounds assigned to unidentified materials or a sound modulated by means of a converted sensor signal.

A modulated sound may be used in a learning process of a user. When said user encounters the same materials time and time again, the same responses will be provided by the system thereby the user will recognize the sounds quicker and correctly associate said sounds with the given material.

### Contact sensor

The tactile sensor 10, which can be a piezoelectric sensor forming the component of the system, is effective when tangential movement across a particular surface takes place. The synaesthetic system allows a possibility of detecting the first contact with the surface, which can be converted into, for example, a short audio signal. It is information for a user, that they touched an object or a surface 16.

Apart from prosthetic solutions, this invention may serve as an extension to perceptual skills of a person. Taking a case in which the tactile sensor 10 has higher sensitivity than sensory neurons in human skin into consideration, the measurement skills of a person should increase. The outcome of the measurement, instead of numerical values or a diagram, takes the form of the sound that can be analyzed by a user on regular basis.

After connecting the system to a loudspeaker, sensing tactile stimuli by means of the sounds is also possible for third parties. It allows for group analysis, which may be an interesting and useful tool at work or for entertainment.

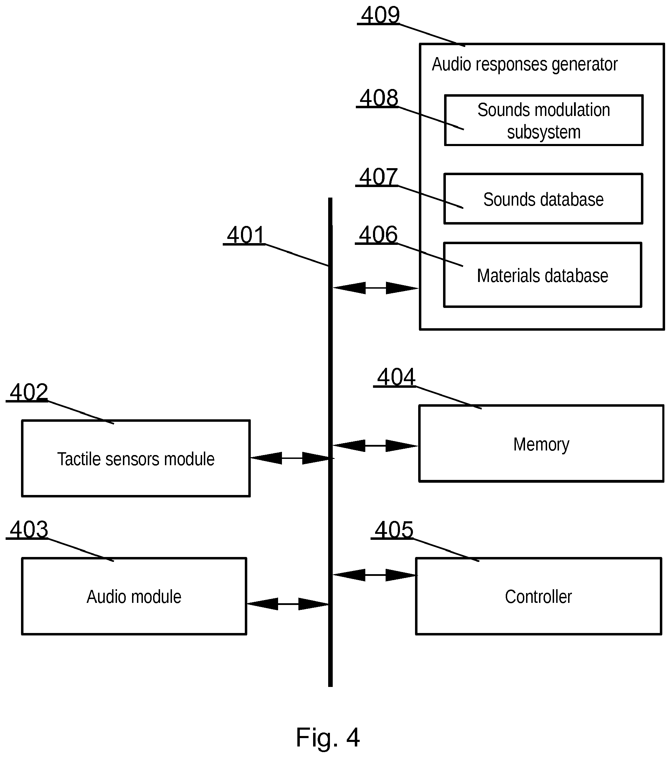

Fig. 4 presents a block diagram of the system according to the present invention. The system may be realized using dedicated hardware components or custom made FPGA (Field-Programmable Gate Array) or ASIC (Application-Specific Integrated Circuit) circuits. The system comprises a data bus 401 communicatively coupled to a memory 404. Additionally, other components of the system are communicatively coupled to the system bus 401 so that they may be managed by a controller 405.

The memory 404 may store computer program or programs executed by the controller 405 in order to execute steps of the method according to the present invention. Further, the memory 404 may store user configuration such as a preferred correspondence between a surface material and an audio clip.

A tactile sensors module 402 is responsible for communication with at least one tactile sensor 10 and providing received data to the controller 405 for analysis. A prosthesis 17 may have only one finger equipped with said tactile sensor 10 or more fingers may have a corresponding tactile sensor 10.

Regarding sound generating options and audio responses generated according to signals received from said tactile sensor 10, three options are possible to implement an audio responses generator 409. Option (A) is having a materials database 406 comprising data identifying materials known to the system and signal parameters with which such materials respond to the at least one tactile sensor 10.

Further, in Option (A), the system comprises a sound database 407 comprising data identifying audio clips available for use.

In Option (B) there is present a sounds modulation subsystem 408 which is configured to generate sound signal modulated by means of a converted tactile sensor's signal as previously described.

The last option of configuration of the audio responses generator 409 is to have both Option (A) and Option (B) implemented, wherein for an unidentified material, a modulated response may be provided (Option (B)), while for an identified material a response with a predefined audio clip may be provided (Option (A)).

An audio module is configured to output a selected audio clip via a give transmission interface on to output sound in case the device comprises a built-in loudspeaker.

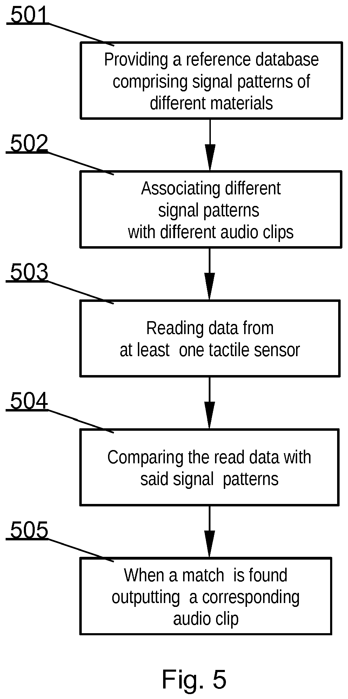

Fig. 5 presents a diagram of the method according to the present invention. The method starts at step 501 from providing a reference database comprising signal patterns of different materials (see module 406). Subsequently, at step 502, the process executes associating different signal patterns with different audio clips (see module 407 and a configuration stored in the memory 404). Next, at step 503, the system executes reading data from at least one tactile sensor 10 and comparing 504 the read data with said signal patterns. When a match is found 505 the system is configured for outputting a corresponding audio clip to a user via said audio module 403.

The present invention facilitates restoration of perceptual skill for persons after a partial amputation of an upper limb. Therefore, the invention provides a useful, concrete and tangible result.

According to the present invention a device is presented which reads data from respective sensors, processes said data and outputs a suitable, tangible result. Thus, the machine or transformation test is fulfilled and that the idea is not abstract.

At least parts of the methods according to the invention may be computer implemented. Accordingly, the present invention may take the form of an entirely hardware embodiment, an entirely software embodiment (including firmware, resident software, micro-code, etc.) or an embodiment combining software and hardware aspects that may all generally be referred to herein as a "circuit", "module" or "system".

Furthermore, the present invention may take the form of a computer program product embodied in any tangible medium of expression having computer usable program code embodied in the medium.

It can be easily recognized, by one skilled in the art, that the aforementioned method for synesthesia may be performed and/or controlled by one or more computer programs. Such computer programs are typically executed by utilizing the computing resources in a computing device. Applications are stored on a non-transitory medium. An example of a non-transitory medium is a nonvolatile memory, for example a flash memory while an example of a volatile memory is RAM. The computer instructions are executed by a processor. These memories are exemplary recording media for storing computer programs comprising computer-executable instructions performing all the steps of the computer-implemented method according the technical concept presented herein.

While the invention presented herein has been depicted, described, and has been defined with reference to particular preferred embodiments, such references and examples of implementation in the foregoing specification do not imply any limitation on the invention. It will, however, be evident that various modifications and changes may be made thereto without departing from the broader scope of the technical concept. The presented preferred embodiments are exemplary only, and are not exhaustive of the scope of the technical concept presented herein.

Accordingly, the scope of protection is not limited to the preferred embodiments described in the specification, but is only limited by the claims that follow.

## Claims

1. A synaesthetic system for converting a signal caused by contact or movement across surfaces into a sound, the system being **characterized in that** it comprises:
- at least one tactile sensor (10) operated by a tactile sensors module (402);
- an audio responses generator (409) configured to generate audio responses according to signals received from said tactile sensor (10);
- a controller (405) configured to
- receive data from said at least one tactile sensor (10);
- receive an audio response from said audio responses generator (409); and
- output said audio response.

2. The synaesthetic system according to claim 1, wherein said at least one tactile sensor (10) is a piezoelectric sensor.

3. The synaesthetic system according to claim 1, wherein said audio responses generator (409) comprises:
- a materials database (406) comprising data identifying materials known to the system and signal parameters with which such materials respond to the at least one tactile sensor (10);
- a sounds database (407) comprising data identifying audio responses;
- a memory (404) configured to store a correspondence between a surface material, from said materials database (406), and an audio clip, from said sounds database (407);
- wherein said controller (405) configured to
- receive data from said at least one tactile sensor (10) and identify presence of a correspondence between said data and a material from said materials database (406); and
- in case such correspondence is found, to output an identified audio response.

4. The synaesthetic system according to claim 3, wherein said materials database (406) comprises, for each material's signal sample, an average voltage, a minimum voltage, a maximum voltage, standard deviation as well as a number of times the voltage crosses zero.

5. The synaesthetic system according to claim 1, wherein said outputting an identified audio response if effected by a wireless communication to a headphones unit.

6. The synaesthetic system according to claim 1, wherein said audio clip is output using a Bluetooth communication.

7. The synaesthetic system according to claim 1, wherein at least one tactile sensor (10) is positioned on a prosthetic (17).

8. The synaesthetic system according to claim 1, wherein said system has a shape of a glove, comprising said tactile sensor (10) at a tip of at least one finger of said glove wherein said central processing unit (13) is sewn in at the wrist of the glove.

9. The synaesthetic system according to claim 1, wherein said system is integrated into a prosthesis.

10. The synaesthetic system according to claim 1, wherein said audio response is modulated by the data from said at least one tactile sensor (10).

11. The synaesthetic system according to claim 10, wherein said modulation process comprises normalization of said data within a predefined range of sound parameters.

12. A synaesthetic method for converting a signal caused by contact or movement across surfaces into a sound, the method being **characterized in that** it comprises the step of:
- providing (501) a reference database comprising signal patterns of different materials (406);
- associating (502) different signal patterns with different audio clips (407, 404);
- reading (503) data from at least one tactile sensor (10) and comparing (504) the read data with said signal patterns;
- wherein when a match is found (505) outputting a corresponding audio clip to a user via an audio module (403).

13. A computer program comprising program code means for performing all the steps of the computer-implemented method according to claim 12 when said program is run on a computer.

14. A computer readable medium storing computer-executable instructions performing all the steps of the computer-implemented method according to claim 12 when executed on a computer.
